# EUROPEAN PATENT SPECIFICATION

(11) **EP 2 105 498 B1**
(45) Date of publication and mention of the grant of the patent: **23.05.2012**
(21) Application number: 09156020.1
(22) Date of filing: 24.03.2009
(51) Int. Cl.: C12N 5/00

(54) **Therapeutic composition for atopic dermatitis**
Therapeutische Zusammensetzung für atopische Dermatitis
Composition thérapeutique pour dermatite atopique

(30) Priority: 28.03.2008 JP 2008088641
(43) Date of publication of application: 30.09.2009
(73) Proprietor: JCR Pharmaceuticals CO., LTD., Ashiya-shi, Hyogo 659-0021 (JP); Miracure Inc., Tokyo 191-0002 (JP)
(72) Inventor: Kuroda, Masahiko, Tokyo Kanagawa 167-0052 (JP); Takanashi, Masakatsu, Kanagawa 259-1141 (JP); Sudo, Katsuko, Saitama 351-0012 (JP); Yamauchi, Shigeki, Kanagawa 211-0065 (JP)
(74) Representative: Isarpatent

(56) References cited:
- WO-A-2005/093044
- WO-A-2008/102937
- WO-A-2008/116157
- SABINE FRANÇOIS ET AL: "Human mesenchymal stem cells favour healing of the cutaneous radiation syndrome in a xenogenic transplant model" ANNALS OF HEMATOLOGY, SPRINGER, BERLIN, DE, vol. 86, no. 1, 17 October 2006 (2006-10-17), pages 1-8, XP019459154 ISSN: 1432-0584

## Description

### FIELD OF THE INVENTION

The present invention relates to a therapeutic composition for use in atopic dermatitis, more specifically to a therapeutic composition for use in atopic dermatitis comprising, as an active principle, mesenchymal stem cells of a mammal, in particular of a human.

### BACKGROUND OF THE INVENTION

IgE, an isotype of immunoglobulin, is produced by such B cells that have been stimulated with an antigen and then switched into IgE class cells, as being specific to the very antigen. IgE molecules thus produced then bind at their Fc region to Fcε receptors present on the surface of mast cells, which occur in subcutaneous as well as connective tissues, or of basophils, and thus stay there. If they come into contact with the same antigen, two of adjacent IgE molecules bind to the antigen falling between them, via which two adjacent Fcε receptors thus become cross-linked. This triggers activation of certain membrane-bound enzymes. This activation results, through degranulation, in an abrupt release of chemical mediators such as histamine that are stored in numerous granules within those cells, as well as in the release of other chemical mediators from the cell membrane, such as leukotrienes, prostaglandins, platelet activating factors, and the like, thereby bringing about a wide range of physiological effects.

Atopy is a diathesis with which production of IgE is readily induced even against those common antigens which generally occur in the environment and induce no particular immune response in healthy individuals. The body of an individual with this diathesis, whether a human or other mammals, is predisposed to recognize as allergens some or other of such factors in the environment that are generally harmless, and thus produces IgE which is specific to them. The IgE thus produced is accumulated on the surface of mast cells and like cells: When it is contacted by the same antigen, release from the cells of histamine and other chemical mediators is triggered. Histamine has such activities as a bronchial smooth muscle contracting activity, a blood vessel permeability enhancing activity, and a mucus secretion inducing activity, which, in combination with the activities of other chemical mediators, bring about a series of allergic reactions, causing damages to various tissue, too (atopic dermatitis, atopic type bronchial asthma, allergic rhinitis, and the like).

Atopic dermatitis, one of atopic diseases, formerly developed in early childhood in most patients, and cured before they grew into adults. In recent year, however, the number of the cases has been increasing in which cure is not attained even though the patient has grown into an adult, and in increasing number of patients, the disease which once cured, recurs in his or her adulthood, or the disease first develops only after the patient has reached his or her adulthood. As a result, the total number of patients with atopic dermatitis has been rapidly increasing; from 318,000 in 1996, to 399,000 in 1999, and at present greatly expanding toward 500,000 (estimated by Ministry of Health, Labor and Welfare).

Atopic dermatitis first develops on the head of the patient in majority of the cases, and then gradually expands to the face, the earlobes, the trunk, and the limbs. Its symptom is chronic eruptions in the skin (rashes), and in accordance with the degree of its progression and the age of the patient, exhibits a wide variety of conditions including, for example, erythema (swelling/edema/infiltration, lichenification), papules (solid, serous), pruriginous nodules, scales (pityroid, foliate, figuratus), crusts (scabs), blisters, pustula, erosion, ulcer, pigmentation, depigmentation, xerosis, and pore cornification, etc., with persistent severe itching. As the disease is aggravated into severe stages, such symptoms are noted as erythema accompanied by severe swelling/edema/infiltration or lichenification, increased formation of papules, severe grades of scales, adhered crusts, small blisters, erosion, a number of scratch wound traces and pruriginous nodules, and thereby the quality of life (QOL) of the patient is heavily impaired.

As atopic dermatitis exhibits severe symptoms like these, the increase in the number of its patient is becoming a social issue. Since a drug therapy has not been found yet that could bring about complete cure of the disease, there is no other choice at present than to resort to symptomatic therapies. The drugs whose efficacy and safety have so far been established for easing inflammation in atopic dermatitis are external steroidal preparations. They are classified according to their potency of effects: for severely affected patients, the first choice is considered to be those external preparations which exhibit most potent effects and thus belong to the so-called "strongest" class (such as clobetasol propionate, diflorasone diacetate), or those which follow next to them and belong to the "very strong" class (such as mometasone furoate, betamethasone butyrate propionate, fluocinonide, betamethasone dipropionate, difluprednate, budesonide, amcinonide, diflucortolone valerate, hydrocortisone butyrate propionate). For patients who are moderately affected, external steroidal preparations of the "strong" class, or those of the "medium" class with moderate potency of effects, are considered to be the first choice, and for patients who are affected only mildly, external steroidal preparations of the "medium" class or less potent ones.

These external steroidal preparations, however, are nothing more than drugs for symptomatic treatment, and therefore they must be used continuously to obtain persistent suppression of atopic dermatitis. And in these drugs, the potency of their activity and that of their side effects generally correlate with each other. For example, if an external steroidal preparation of the "strongest" class is applied at high doses and for an extended length of time, side effects will sometimes result due to the systemic absorption of the drug, such as suppression of the functions of the pituitary-adrenal cortex system or induction of the development of cataract or glaucoma. Furthermore, suppression of cell growth by steroids could cause such disorders in the skin area to which they are applied for an extended length of time, as dermatrophy, striae cutis distensae, dryness, ichthyosis-like lesion, retardation of wound healing, steroid purpura, telangiectasis, dyschromatosis, rosacea-like dermatitis, etc. For theses reasons, it sometimes is difficult to continuously apply external steroidal preparations for an extended period of time. It also is a problem that rebound would occur when application of them is disrupted after an extended period of use. Furthermore, not a small number of people mistrust steroids, and they, avoiding external steroidal preparations, have failed to receive even a necessary and proper treatment until their conditions become severe, thereby suffering a great disadvantage.

Thus, while the number of the patients has been great and even increasing further, there has so far been no such therapeutic composition for atopic dermatitis that is sufficient in both aspects of efficacy and safety. Thus, development of a new therapeutic composition for the treatment of atopic dermatitis has been longed for.

Mesenchymal stem cells (MSC) are multipotent stem cells which occur in a very small number in bone marrow and other mesenchymal tissues. They have high potential to proliferate and are capable of differentiating into osteocytes, chondrocytes, muscle cells, tendon cells, stromal cells, adipose cells, and the like. It is known that human mesenchymal stem cells can be cultured in an artificial medium to let them proliferate (see Patent Document 1), and, like other cultured cells, can be stored and supplied in a frozen state. It is known, too, that mesenchymal stem cells can be obtained not only from the bone marrow but also from various other tissues such as adipose tissue (see Patent Document 2), dental pulp tissue (see Patent Document 3), placenta tissue, umbilical cord tissue (see Patent Document 4), and the like.

Disclosures have been made that rejection of an allogeneic skin graft between baboons is suppressed in the recipient which have been administered with baboon mesenchymal stem cells; that graft-versus-host disease (GvHD) in a dog after allogeneic transplantation of bone marrow is suppressed by administration of canine mesenchymal stem cells; that human T lymphocytes do not show proliferative response to human (allogeneic) mesenchymal stem cells in vitro (mixed lymphocyte reaction); that human mixed lymphocyte reaction is suppressed non-specifically to MHC type by human mesenchymal stem cells, and the same is observed in dogs; and, on the basis of these results, about the possibility of applying human mesenchymal stem cells for suppression of immune responses after transplantation between humans (see Patent Documents 5, 6, which derive from a common priority application, and Patent Document 7, which is a translation of the latter).

Expecting such effects of mesenchymal stem cells as mentioned above, a clinical trial was conducted in the United States on steroid-resistant severe GvHD patients to evaluate human mesenchymal stem cell's suppressive effect on acute graft-versus-host disease (GvHD) in human following bone marrow transplantation, and the result has been reported (see Non-patent Documents 1 and 2). According to the report, the patients had been treated in advance with anticancer compositions (cyclophosphamide, busulphan, fludarabin) and/or with total body irradiation (TBI) against their malignant tumors, and were administered with methotrexate, cyclophosphamide, or prednisolone for prevention and treatment of GvHD, and, under those conditions, the patients were administered with human mesenchymal stem cells (0.7-9 × 10⁶ cells/kg body weight). The administration of the human mesenchymal stem cells to the patients, who had their immune system suppressed, was reported to have induced a response in six out of the eight patients as a whole.

In connection with GvHD, though mesenchymal stem cells are thought to bring about T cells-mediated suppression of the immune cells of the host (see Non-patent Document 3), the clear mechanism of it is yet to be elucidated.

It is also proposed that mesenchymal stem cells be used to treat neovascularization, autoimmune diseases, inflammatory responses (in Alzheimer disease, Parkinson's disease, cerebral stroke, brain-cell lesions, psoriasis, chronic dermatitis, contact dermatitis, arthrosteitis, arthritis including rheumatoid arthritis, inflammatory bowel disease, chronic hepatitis), cancer, allergic diseases, sepsis, trauma (burn, surgery, transplantation), inflammation of various tissues or organs (cornea, lens, pigment epithelium, retina, brain, spinal cord, uterus during pregnancy, ovary, testis, adrenal gland) (see Patent Document 8). However, in this document, the functions of mesenchymal stem cells were examined in vitro only, and no examination was conducted either in vivo or in a model of any of those particular diseases. Therefore, the document does not provide any result which could serve as a key to evaluating usefulness of mesenchymal stem cells for such diseases.
[Patent Document 1] United States Patent 5486359
[Patent Document 2] Japanese Patent Application Publication 2004-129549
[Patent Document 3] Japanese Patent Application Publication 2004-201612
[Patent Document 4] Japanese Patent Application Publication 2004-210713
[Patent Document 5] United States Patent 6328960
[Patent Document 6] WO 99/47163
[Patent Document 7] Japanese Patent Application Publication 2002-50683
[Patent Document 8] WO 2005/093044
[Non-patent Document 1] Transplantation. 2006; 81(10): 1390-7
[Non-patent Document 2] Br J Haematol. 2007; 137(2): 87-98
[Non-patent Document 3] Blood. 2005; 105(4): 1815-22

### SUMMARY OF THE INVENTION

Under the above circumstances, the objective of the present invention is to provide a non-steroidal, novel type of composition for the treatment of atopic dermatitis.

### BRIEF DESCRIPTION OF THE FIGURES

The present inventors injected atopic dermatitis model mice intravenously with human mesenchymal stem cells, and followed their course of disease. It was found that the mice did not exhibit any abnormal reactions even though the injected cells were heterologous to them, and that not only the progress of their dermatitis was greatly suppressed after just a limited times of administration, but also the condition of their skin rapidly improved toward normalization. The present invention was completed based upon those findings.

Thus, the present invention provides what follows:
1. A composition for use in the treatment of atopic dermatitis comprising human mesenchymal stem cells as the active principle.
2. The composition for use in the treatment of atopic dermatitis according to 1 above, wherein the atopic dermatitis is that which has developed in a mammal.
3. The composition for use in the treatment of atopic dermatitis according to 2 above, wherein the mammal is selected from the group consisting human, dog, cat, rabbit, and rodent.
4. The composition for use in the treatment of atopic dermatitis according to one of 1 to 3 above, wherein the human mesenchymal stem cells are of human bone-marrow origin.
5. The composition for use in the treatment of atopic dermatitis according to 4 above, wherein the mammal is a human and is not the same as the human from whom the human mesenchymal stem cells originate.
6. The composition for use in the treatment of atopic dermatitis according to one of 1 to 5 above, wherein the composition is an injection.
7. The composition for use in the treatment of atopic dermatitis according to 6 above, wherein the composition is an injection for intravenous administration.
8. A human mesenchymal stem cell for use as a medicament for the treatment of atopic dermatitis.
9. The human mesenchymal stem cell use in according to 8 above, wherein the atopic dermatitis is mammal's atopic dermatitis.
10. The human mesenchymal stem cell for use according to 9 above, wherein the mammal is selected from the group consisting of human, dog, cat, rabbit, and rodent.
11. The human mesenchymal stem cell for use according one of 8 to 10 above, wherein the human mesenchymal stem cell is of human bone-marrow origin.
12. The human mesenchymal stem cell for use according to one of 8 to 11 above, wherein the mammal is a human.
13. The human mesenchymal stem cell for use according to 12 above, wherein the human is not the same as the human from whom the mesenchymal stem cell originates.
14. A method for the treatment of atopic dermatitis in a patient comprising administering to the patient a therapeutically effective amount of human mesenchymal stem cells.
15. The method for the treatment of atopic dermatitis in a patient according to 14 above, wherein the patient is a mammal.
16. The method for the treatment of atopic dermatitis in a patient according to 14 or 15 above, wherein the mammal is selected from the group consisting of human, dog, cat, rabbit, and rodent.
17. The method for the treatment of atopic dermatitis in a patient according to one of 14 to 16 above, wherein the human mesenchymal stem cells are of human bone-marrow origin.

Human mesenchymal stem cells, and therefore the composition for use in the treatment of atopic dermatitis according to the present invention, can not only remarkably suppress the progression of atopic dermatitis in a mammal, but also greatly improve the condition of the skin. Further, they exhibit such effects after only a very limited number of rounds of their administration, and persistently thereafter. Human mesenchymal stem cells and the composition for use in the treatment of atopic dermatitis according to the present invention, therefore, can be used with great advantages for the treatment of atopic dermatitis in a human or a pet (dog, cat, rodent, rabbit, or the like).

Thus, present invention also provides use of human mesenchymal stem cells for the production of a composition for use in the treatment of atopic dermatitis in a patient, as well as a method for the treatment of atopic dermatitis in a patient, e.g., mammal, such as human, dog, cat, rabbit, rodent, and the like.

### BRIEF DESCRIPTION OF THE FIGURES

Fig. 1 is a photograph showing the general appearance of an atopic dermatitis model mouse that has developed score 4 dermatitis.
Fig. 2 is a photograph showing the general appearance of a mouse before developing dermatitis.
Fig. 3 is a graph showing the profiles of the dermatitis scores in the MSC-administered and the control groups.

### DETAILED DESCRIPTION OF THE INVENTION

The composition for use in the treatment of atopic dermatitis according to the present invention comprises human mesenchymal stem cells as the active principle (active agent).

In the present invention, any human mesenchymal stem cells may be employed that are taken from any tissue including bone marrow, adipose tissue, dental pulp tissue, placenta tissue, etc. Preferable, however, are those originating from human bone marrow. Mesenchymal stem cells which were obtained from human bone marrow and formed into a cell line are already on the market (Poetics^{™}, Cambrex Bio Science Walkersville, Inc., MD, USA), and may be employed, either directly or after further subcultivation.

In the present invention, it is not necessary for the human mesenchymal stem cells to be of a primary culture, but subcultured cells may be employed. While primary culture cells may be used directly, those usually used may be subcultured cells, considering the actual requirements for convenience in their production through cell growth, as well as their storage and use, in a substantial amount. In the present invention, the active principle, human mesenchymal stem cells, may be supplied, for example, in a frozen state, and thawed just before their administration, suspended in an aqueous medium, and then administered. However, doing so is not a prerequisite and, for example, human mesenchymal stem cells collected from a culture and not having undergone freezing, may be used directly in the form of a suspension. In doing this, an aqueous medium in which to suspend the cells may be, for example, an injectable aqueous solution which has its osmotic pressure, pH, and salts concentration, and the like adjusted substantially to those of the blood. Intravenous fluids such as acetated Ringer's solution, glucose acetated Ringer's solution, etc., physiological saline, or glucose solution are non-limiting examples which may be used. For example, to Ringer's solution for intravenous infusion which is to be used, an acceptable amount of dimethylsulfoxide (DMSO) or human serum albumin (HSA) may be added.

In the present invention, human mesenchymal stem cells may be administered in the form of an injectable preparation, whether intravenously, intramuscularly, or subcutaneously, etc., among which intravenous administration is preferred. When conducting intravenous administration, the injectable preparation may be administered either directly from the syringe to the patient, or dropwise from a drip bag containing the intravenous fluid to which its has been added beforehand. Furthermore, human mesenchymal stem cells may be injected directly in the affected area of the skin.

As to the administration regimen of the composition according to the present invention, administration may be made only once, or it may be made multiple times, e.g., twice, thrice or more, until the effect of composition is observed, according to the severity of the symptoms of atopic dermatitis. However, since the composition according to the present invention exhibits remarkable persistent effect after administration, and, further, as the effect of the composition according to the present invention is thought to consist in inducing autonomous normalization of the behavior of the patient's own immune system in the skin, no additional administration of the composition will be needed as long as the inflammation has been ameliorated and the condition is continuing.

The composition for the treatment of atopic dermatitis according to the present invention is a therapeutic composition commonly applicable to any patient, and thus it will be administered, in general, to patients who are allogeneic to the origin of the human mesenchymal stem cells contained as the active principle. However, its administration to an syngeneic patient (e.g., one of the homozygotic twins the other of whom is the origin of the mesenchymal stem cells) is also permitted. Further, it is also allowed that the composition for the treatment of atopic dermatitis according to the present invention, which has, through the process of cell growth in culture, been prepared as a commonly applicable composition, is administered by chance to the very patient who is the origin of the mesenchymal stem cells.

When administered, the density of the human mesenchymal stem cells in the composition according to the present invention may be preferably 1 × 10² - 1 × 10⁹ cells/mL, and more preferably 1 × 10³ - 1 × 10⁸ cells/mL. And the number of the cells to be administered to a human at one time may, in general, be 1 × 10⁵ - 1 × 10⁷ cells/kg body weight, though it depends on the intended frequency of administration. However, these are not a prerequisite, and those numbers may altered as desired in accordance with the degree of the symptoms. Particularly, according to the findings that the inventors have already obtained separately through administration of mesenchymal stem cells to arthritis model animals (Japanese Patent Application No. 2007-233094, not yet laid open), human mesenchymal stem cells administered to an animal homed to the site of inflammation, and, therefore, even if the number of human mesenchymal stem cells administered is relatively small, they accumulate at the site of inflammation and exhibit an excellent effect there. Also, human mesenchymal stem cells, when they are no longer required, will vanish away by themselves. Thus, the dose of human mesenchymal stem cells may be set as desired in a wide range.

### EXAMPLE 1

The present invention will be described in further detail below with reference to examples. However, it is not intended that the present invention be limited to those examples. The method for making atopic dermatitis model mice, the schedule of administration of mesenchymal stem cells, and the method of evaluation of dermatitis are presented below.

### [Method for making atopic dermatitis model mice]

NC/Nga mice are mice of a pure strain that have been derived from an indigenous Japanese mice called "Nishiki Nezumi". NC/Nga mice will not develop dermatitis if maintained under an SPF (Specific Pathogen Free) condition. However, if they are maintained under a conventional, i.e., a non-SPF condition, they will develop dermatitis. The dermatitis thus developed appears, both clinically and histologically, very similar to human atopic dermatitis. Namely, the mice exhibit the symptom of strong itchiness, rougher body hair, especially on the head and the back, erythema, eczema-like lesions, hemorrhage, crusts, excoriation, erosions, scaling and dryness, which occur everywhere in the body surface including the ears, nose, and dorsal skin. NC/Nga mice maintained under a non-SPF condition have higher blood IgE values than those maintained under a SPF condition, and exhibit infiltration of numerous eosinophils in the skin, increase in number of mast cells, and degranulation by their activation. The symptoms will progress and become severe along with the weeks of age, which sometimes goes to the loss of the earlobe (Int. Arch. Allergy Immunol., 120 (Supple. 1), 70-75). Dermatitis similar to atopic dermatitis is also inducible in these mice, by letting mites parasitize them, or also by application of a drug such as picryl chloride (Biol. Pharm. Bull. 28(1) 78-82 (2005)), and thus they are used as atopic dermatitis model mice.

Male, four-week old NC/Nga mice (Japan Slc, Inc., Hamamatsu, Shizuoka) were purchased and maintained for two weeks under a SPF condition for acclimatization before they were used in the test. Throughout this period of acclimatization and testing, they were maintained under the condition where lighting was on and off at 12-hour intervals, and the temperature was kept constant, with γ-irradiated food and water provided, to which the mice was accessible ad libitum. The method for keeping them complied with a relating rule of Experimental Animals Center, Tokyo Medical University. After the acclimatization, the mice were parasitized by Myobia musculi, and maintained for further 10-12 weeks to develop spontaneous dermatitis. The general condition of the mice were observed, and the severity of their dermatitis was evaluated according to the criterion for evaluation of severity of dermatitis as shown in Table 1. Six mice whose dermatitis symptoms were graded as score 2 (moderate) were taken out and divided into two groups, thee mice each.

**Table 1. Criterion for Evaluation of Severity of Dermatitis**

| | Score | Symptoms |
|---|---|---|
| 0 | (normal) | no dermatitis, or complete cure |
| 1 | (mild) | very mild erythema, mild wound, or cure to comparable grade |
| 2 | (moderate) | manifest erythema, wound, mild hemorrhage, or cure to comparable grade |
| 3 | (severe) | sever erythema, wound (partial loss of earlobe), localized ulcer, crust formation, or cure to comparable grade |
| 4 | (loss of earlobe) | much hemorrhage, or loss of earlobe with hemorrhage, wide range of ulcer |

### [Administration of human mesenchymal stem cells]

In one (MSC administered group, n=3) of the two groups of atopic dermatitis model mice prepared above, the mice were administered with 1 × 10⁶ cells of human mesenchymal stem cells (Poietics^{™}, Cambrex: bone marrow-derived, CD105⁺, CD166⁺, CD29⁺, CD44⁺, CD14⁻, CD34⁻, CD45⁻), which had been suspended in 0.21 mL of acetated Ringer's solution for intravenous infusion fluid (PlasmaLyteA:Baxter) containing 3.7 % DMSO and 5% HSA (human serum albumin) and been kept at room temperature, through tail vein at 1 mL/min. After the mice was warmed over a warm bath to let the vein dilate, the administration was carried out to constrained mice put in a fixation device. Following the first administration of human mesenchymal stem cells (day 1), administration of human mesenchymal stem cells was repeated in the same manner also on days 2 and 3. In the other group (Control group, n=3), the mice received, in the same manner as above, 0.21 mL of acetated Ringer's solution for intravenous infusion fluid containing 3.7 % DMSO and 5% HSA, which was free of human mesenchymal stem cells.

### [Evaluation of dermatitis]

Table 1. Evaluation was made everyday, from day 1 through day 10. The dermatitis score for each animal and the mean score for each group is given in Table 2.

**Table. 2 Dermatitis Score**

| Control group | | | | | MSC administered group | | | | |
|---|---|---|---|---|---|---|---|---|---|
| | No. 1 | No. 2 | No. 3 | Mean | | No. 1 | No. 2 | No. 3 | Mean |
| Day 1 | 2 | 2 | 2 | 2 | Day 1 | 2 | 2 | 2 | 2 |
| Day 2 | 3 | 3 | 3 | 3 | Day 2 | 3 | 3 | 3 | 3 |
| Day 3 | 4 | 3 | 4 | 4 | Day 3 | 3 | 3 | 3 | 3 |
| Day 4 | 4 | 4 | 4 | 4 | Day 4 | 3 | 3 | 3 | 3 |
| Day 5 | 4 | 4 | 4 | 4 | Day 5 | 3 | 3 | 3 | 3 |
| Day 6 | 4 | 4 | 4 | 4 | Day 6 | 3 | 3 | 3 | 3 |
| Day 7 | 4 | 4 | 4 | 4 | Day 7 | 3 | 3 | 2 | 3 |
| Day 8 | 4 | 4 | 4 | 4 | Day 8 | 2 | 2 | 2 | 2 |
| Day 9 | 4 | 4 | 4 | 4 | Day 9 | 2 | 2 | 1 | 2 |
| Day 10 | 4 | 4 | 4 | 4 | Day 10 | 1 | 2 | 1 | 1 |

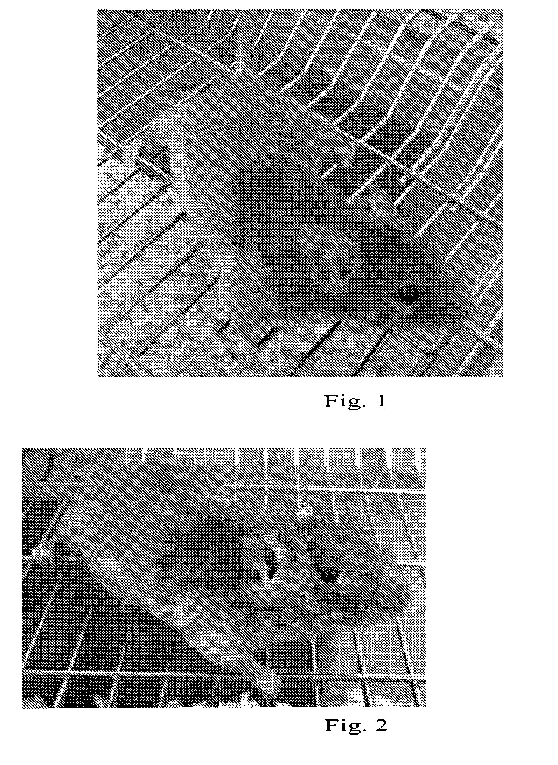

In both groups, the dermatitis score was 2 for all the animals on day 1. In the control group, however, on day 2 (at the second administration), the score was 3 for all the animals; on day 3, one of the three animals had score 3 but the other two had the highest score of 4; and on day 4, all the animals had score 4, resulting in severe dermatitis. As a result, in the control group, there were observed much hemorrhage from the earlobe or partial loss of the earlobe with hemorrhage, and ulcer ranging from the head, face, earlobe to trunk and forelimbs. All the animals in the control group subsequently constantly exhibited the severest, score 4 dermatitis. Fig. 1 shows the general appearance of a mouse before the development of dermatitis, and Figs. 2 the general appearance of mouse No. 1 in the control group, on day 3, respectively.

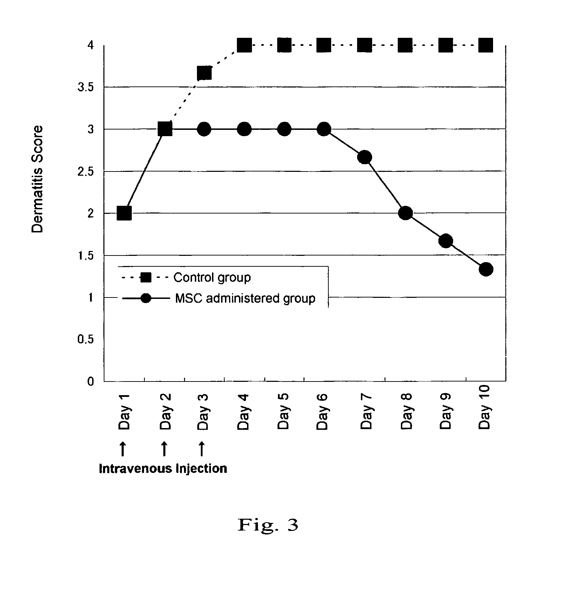

In contrast, though the severity of dermatitis in the MSC administered group was graded as score 2 for all the animals on day 1, and then score 3 for all the animals on day 2, no aggravation to score 4 dermatitis occurred thereafter in any of the animals, and, on the contrary, since 4 days after the last administration (day 3) of MSC, start of remarkable improvement of the skin condition was observed. Namely, the severity of dermatitis in all the animals of the MSC administered group stayed at score 3 from day 4 through day 6; the score then was improved to 2 in one of the three animals on day 7; score 2 in all the three animals on day 8; score 1, which was lower than the score recorded when MSC administration was started, in one of the three animals on day 9; and score 1 in also one of the remaining two animals on day 10. Fig. 3 shows a graphic illustration of the profile mean value of the scores for each group.

As evident from the results, in MSC-administered animals, not only formation of severe dermatitis was prevented, but also rapid improvement of the skin condition followed, indicating that administration with human mesenchymal stem cells exhibited remarkable therapeutic effect on atopic dermatitis. Especially, the skin condition remarkably improved after the administration of MSC on day 1 through day 3 was over, i.e., 4 days after the termination of MSC administration (thus, from day 7 on), which strongly suggests that the administered MSC acted, by some mechanism, to autonomously normalize the behavior of the immune system in the skin. Meanwhile, nothing abnormal was observed in general appearance, such as an adverse immune reaction, in any of the mice of the MSC group, which received repeated (thee times) administration of the heterologous, human MSC.

### Preparation Example 1 Injection

| | |
|---|---|
| Human mesenchymal stem cells (of bone-marrow origin) | 5 × 10⁷ cells |
| Acetated Ringer's solution | to 5 mL |

Human mesenchymal stem cells are suspended in acetated Ringer's solution, and the total volume of the suspension is adjusted to 5 mL to make an injection.

### Preparation Example 2 Injection

| | |
|---|---|
| Human mesenchymal stem cells (of bone-marrow origin) | 1 × 10⁸ cells |
| Sterilized physiological saline | to 10 mL |

Human mesenchymal stem cells are suspended in sterilized physiological saline, and the total volume of the suspension is adjusted to 10 mL to make an injection.

### Preparation Example 3 Injection

| | |
|---|---|
| Human mesenchymal stem cells (of bone-marrow origin) | 2 × 10⁶ cells |
| 5% glucose solution | to 2 mL |

Human mesenchymal stem cells are suspended in 5% glucose solution, and the total volume of the suspension is adjusted to 2 mL to make an injection.

### INDUSTRIAL APPLICABILITY

The composition for the treatment of atopic dermatitis according to the present invention, which contains human mesenchymal stem cells as the active agent, can not only suppress the progression of atopic dermatitis in a mammal but also can remarkably improve the condition of the skin, and, further, persistently achieves the effect after only a very limited number of rounds of administration. Therefore, the active agent is highly useful as a novel type of therapeutic agent for the treatment of atopic dermatitis in a human or a mammalian pet.

## Claims

1. A composition for use in the treatment of atopic dermatitis comprising human mesenchymal stem cells, wherein the composition is to be administered intravenously to a patient with atopic dermatitis.

2. The composition for use according to claim 1, wherein the patient is a mammal including a human, dog, cat, rabbit, and rodent.

3. The composition for use according to one of claims 1 or 2, wherein the human mesenchymal stem cells are of human-bone marrow origin.

4. The composition for use according to claim 2, wherein the mammal is a human and is not the same as the human from whom the human mesenchymal stem cells originate.

5. The composition for use according to one of claims 1 to 4, wherein the composition comprises an injectable aqueous solution in which the stem cells are suspended.

6. The composition for use according to claim 5, wherein the injectable aqueous solution is Ringer's solution.

7. The composition for use according to claim 6, wherein the injectable aqueous solution is Ringer's solution to which dimethylsulfoxide and/or human serum albumin is added.

8. A human mesenchymal stem cell for use in the treatment of atopic dermatitis, wherein the human mesenchymal stem cell is to be administered intravenously.

9. The human mesenchymal stem cell for use according to claim 8, wherein the atopic dermatitis is mammal's atopic dermatitis.

10. The human mesenchymal stem cell for use according to claim 9, wherein the mammal is selected from the group consisting of human, dog, cat, rabbit, and rodent.

11. The human mesenchymal stem cell for use according to one of claims 8 to 10, wherein the human mesenchymal stem cell is of human bone-marrow origin.

12. The human mesenchymal stem cell for use according to one of claims 8 to 11, wherein the mammal is a human.

13. The human mesenchymal stem cell for use according to claim 12, wherein the human is not the same as the human from whom the mesenchymal stem cell originates.

## Patentansprüche

1. Zusammensetzung zur Verwendung in der Behandlung eines atopischen Ekzems umfassend menschliche mesenchymale Stammzellen, wobei die Zusammensetzung intravenös einem Patienten mit einem atopischen Ekzem verabreicht wird.

2. Zusammensetzung zur Verwendung gemäß Anspruch 1, wobei der Patient ein Säugetier einschließlich eines Menschen, Hund, Katze, Kaninchen und Nagetier ist.

3. Zusammensetzung zur Verwendung gemäß einem der Ansprüche 1 oder 2, wobei die menschlichen mesenchymalen Stammzellen aus dem menschlichen Knochenmark stammen.

4. Zusammensetzung zur Verwendung gemäß Anspruch 2, wobei das Säugetier ein Mensch ist, und nicht derselbe wie der Mensch, von dem die menschlichen mesenchymalen Stammzellen stammen.

5. Zusammensetzung zur Verwendung gemäß einem der Ansprüche 1 bis 4, wobei die Zusammensetzung eine injizierbare wässrige Lösung umfasst, in der die Stammzellen suspendiert sind.

6. Zusammensetzung zur Verwendung gemäß Anspruch 5, wobei die injizierbare wässrige Lösung eine Ringerlösung ist.

7. Zusammensetzung zur Verwendung gemäß Anspruch 6, wobei die injizierbare wässrige Lösung eine Ringerlösung ist, zu der Dimethylsulfoxid und/oder humanes Serum-Albumin zugefügt ist.

8. Menschliche mesenchymale Stammzelle zur Verwendung in der Behandlung eines atopischen Ekzems, wobei die menschliche mesenchymale Stammzelle intravenös verabreicht wird.

9. Menschliche mesenchymale Stammzelle zur Verwendung gemäß Anspruch 8, wobei das atopische Ekzem ein Säugetier-atopisches Ekzem ist.

10. Menschliche mesenchymale Stammzelle zur Verwendung gemäß Anspruch 9, wobei das Säugetier ausgewählt ist aus der Gruppe, bestehend aus Mensch, Hund, Katze, Kaninchen und Nagetier.

11. Menschliche mesenchymale Stammzelle zur Verwendung gemäß einem der Ansprüche 8 bis 10, wobei die menschliche mesenchymale Stammzelle aus dem menschlichen Knochenmark stammt.

12. Menschliche mesenchymale Stammzelle zur Verwendung gemäß einem der Ansprüche 8 bis 11, wobei das Säugetier ein Mensch ist.

13. Menschliche mesenchymale Stammzelle zur Verwendung gemäß Anspruch 12, wobei der Mensch nicht derselbe wie der Mensch ist, von dem die mesenchymale Stammzelle stammt.

## Revendications

1. Composition à utiliser pour le traitement de la dermite atopique, comprenant des cellules souche de mésenchyme humain, la composition étant destinée à l'administration intraveineuse à un patient atteint de dermite atopique.

2. Composition à utiliser selon la revendication 1, le patient étant un mammifère parmi lesquels un être humain, un chien, un chat, un lapin et un rongeur.

3. Composition à utiliser selon l'une des revendications 1 ou 2, les cellules souche de mésenchyme humain étant issues de la moelle osseuse humaine.

4. Composition à utiliser selon la revendication 2, le mammifère étant un être humain qui n'est pas le même que celui dont sont issues les cellules souche de mésenchyme humain.

5. Composition à utiliser selon l'une des revendications 1 à 4, la composition comprenant une solution aqueuse injectable dans laquelle les cellules souche sont en suspension.

6. Composition à utiliser selon la revendication 5, la solution aqueuse injectable étant le liquide de Ringer.

7. Composition à utiliser selon la revendication 6, la solution aqueuse injectable étant le liquide de Ringer auquel a été ajouté du diméthylsulfoxide et/ou de la sérumalbumine humaine.

8. Cellule souche de mésenchyme humain à utiliser pour le traitement de la dermite atopique, la cellule souche de mésenchyme humain devant être administrée par voie intraveineuse.

9. Cellule souche de mésenchyme humain à utiliser selon la revendication 8, la dermite atopique étant une dermite atopique du mammifère.

10. Cellule souche de mésenchyme humain à utiliser selon la revendication 9, le mammifère étant choisi parmi le groupe composé de l'être humain, du chien, du chat, du lapin et des rongeurs.

11. Cellule souche de mésenchyme humain à utiliser selon l'une des revendications 8 à 10, la cellule souche de mésenchyme humain étant issue de la moelle osseuse humaine.

12. Cellule souche de mésenchyme humain à utiliser selon l'une des revendications 8 à 11, le mammifère étant un être humain.

13. Cellule souche de mésenchyme humain à utiliser selon la revendication 12, l'être humain n'étant pas le même que celui dont est issue la cellule souche de mésenchyme humain.
